(19) Europäisches Patentamt
European Patent Office
Office européen des brevets

(11) Publication number: 0 356 866 A2

# (12) EUROPEAN PATENT APPLICATION

(21) Application number: 89115400.7

(22) Date of filing: 21.08.89

(51) Int. Cl.[5]: C07F 9/38 , A61K 31/66 , C07F 9/40 , C07F 9/44

(30) Priority: 29.08.88 US 237940

(43) Date of publication of application: 07.03.90 Bulletin 90/10

(84) Designated Contracting States: DE FR GB IT

(71) Applicant: E.R. SQUIBB & SONS, INC.
Lawrenceville-Princeton Road
Princeton New Jersey 08543-4000(US)

(72) Inventor: Sofia, Michael J.
502 White Pine Circle
Lawrenceville New Jersey(US)
Inventor: Biller, Scott A.
136 Nancy Lane
Ewing New Jersey(US)

(74) Representative: Vannini, Torquato et al
JACOBACCI-CASETTA & PERANI S.p.A. 7 Via Visconti di Modrone
I-20122 Milan(IT)

## (54) Phosphorus-containing squalene synthetase inhibitors and method.

(57) Compounds which are useful as inhibitors of cholesterol biosynthesis and thus as hypocholesterolemic agents are provided which have the structure

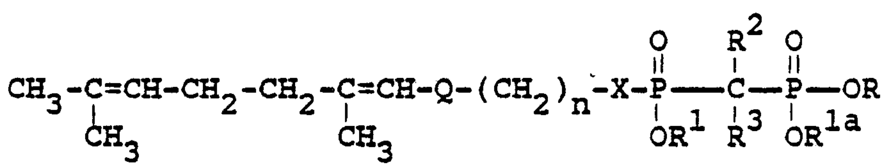

wherein Q is

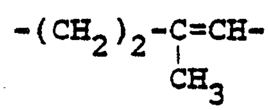

or a bond;
n is 1 to 4; X is O, -NH- or $-NR^4-$;
R, $R^1$ and $R^{1a}$ are the same or different and are H, lower alkyl, lower alkenyl, or a metal ion;
$R^2$ and $R^3$ may be the same or different and are H or halogen; and
$R^4$ is lower alkyl;
with the proviso that when X is oxygen, n is 2, 3, or 4.
A method for using such compounds to inhibit cholesterol biosynthesis is also provided.

EP 0 356 866 A2

## PHOSPHORUS-CONTAINING SQUALENE SYNTHETASE INHIBITORS AND METHOD

The present invention relates to new phosphorus-containing compounds which inhibit the activity of squalene synthetase and thus are useful in inhibiting cholesterol biosynthesis, to hypocholesterolemic compositions containing such compounds and to a method of using such compounds for inhibiting cholesterol biosynthesis.

Squalene synthetase is a microsomal enzyme which catalyzes the reductive dimerization of two molecules of farnesyl pyrophosphate (FPP) in the presence of nicotinamide adenine dinucleotide phosphate (reduced form) (NADPH) to form squalene (Poulter, C. D.; Rilling, H. C., in "Biosynthesis of Isoprenoid Compounds", Vol. I, Chapter 8, pp. 413-441, J. Wiley and Sons, 1981 and references therein). This enzyme is the first committed step of the de novo cholesterol biosynthetic pathway. The selective inhibition of this step should allow the essential pathways to isopentenyl tRNA, ubiquinone, and dolichol to proceed unimpeded. Squalene synthetase, along with HMG-CoA reductase has been shown to be down-regulated by receptor mediated LDL uptake (Faust, J. R.; Goldstein, J. L.; Brown, M. S. Proc. Nat. Acad. Sci. USA, 1979, 76, 5018-5022), lending credence to the proposal that inhibiting squalene synthetase will lead to an up-regulation of LDL receptor levels, as has been demonstrated for HMG-CoA reductase, and thus ultimately should be useful for the treatment and preventing of hypercholesterolemia and atherosclerosis.

One approach to inhibitors of squalene synthetase is to design analogs of the substrate FPP. It is clear from the literature that the pyrophosphate moiety is essential for binding to the enzyme. However, such pyrophosphates are unsuitable as components of pharmacological agents due to their chemical and enzymatic lability towards allylic C-O cleavage, as well as their susceptibility to metabolism by phosphatases.

P. Ortiz de Montellano et al in J. Med. Chem., 1977, 20, 243-249 describe the preparation of a series of substituted terpenoid pyrophosphate (Table A), and have shown these to be competitive inhibitors of the squalene synthetase enzyme. These substances retain the unstable allylic pyrophosphate moiety of FPP.

### Table A

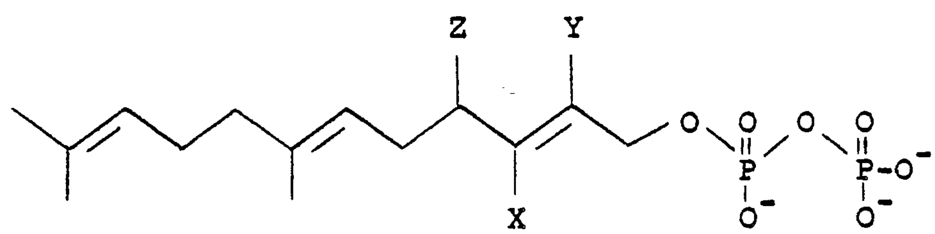

| No. | X | Y | Z |
|---|---|---|---|
| 1 | $CH_3$ | $CH_3$ | H |
| 2 | H | H | H |
| 3 | $C_2H_5$ | H | H |
| 4 | I | H | H |
| 5 | H | I | H |
| 6 | $CH_3$ | H | $SCH_3$ |

Corey and Volante, J. Am. Chem. Soc. 1976, 98, 1291-3, have prepared FPP analog A and presqualene pyrophosphate (PSQ-PP) analog B as inhibitors of squalene biosynthesis. (Presqualene pyrophosphate is an intermediate in the conversion of FPP to squalene). These inhibitors possess methylene groups in place of the allylic oxygen moiety of FPP and PSQ-PP, but still retain the chemically and enzymatically unstable pyrophosphate linkage.

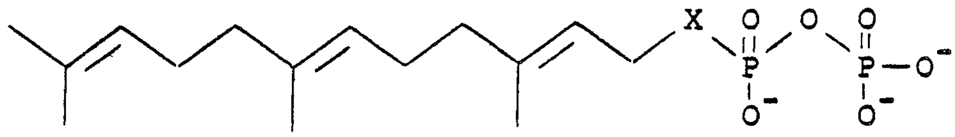

A X = $CH_2$
FPP X = O

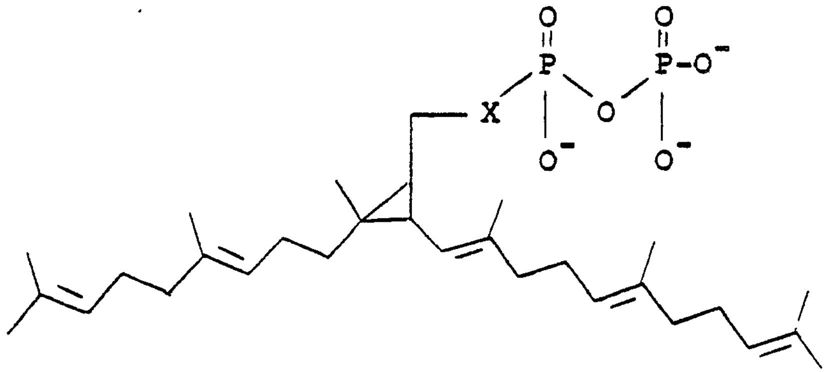

B X = $CH_2$
PSQ-PP X = O

Poulter and co-workers have prepared cyclopropane C (Sandifer, R. M., et al., J. Am. Chem. Soc. 1982, 104, 7376-8) which in the presence of inorganic pyrophosphate is an intermediate analog inhibitor of the enzyme squalene synthetase.

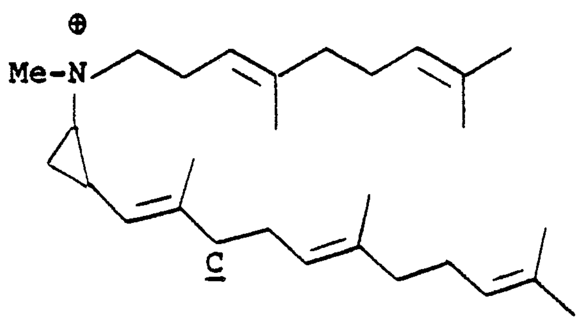

C

Altman and co-workers, Bertolino, A., et al., Biochim. Biophys. Acta. 1978, 530, 17-23, reported that farnesyl amine and related derivatives D inhibit squalene synthetase, but provide evidence that this inhibition is non-specific and probably related to membrane disruption.

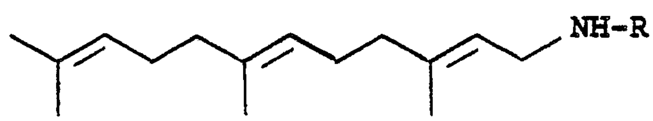

R = H, $CH_2CH_2OH$, $CH_2CH_2OCH_3$

D

Poulter, C.D., et al, J. Org. Chem., 1986, 51, 4768, prepared compound E in a demonstration of a synthetic method, but did not report any biological data.

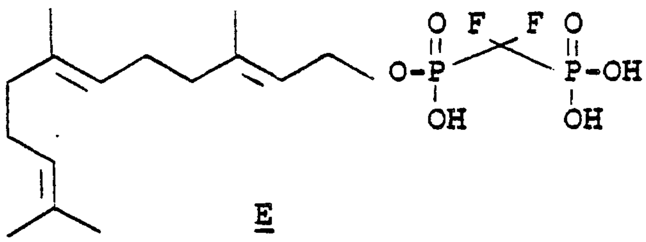

E

Poulter, C.D., Stremler, K.E., J.A.C.S., 1987, 109, 5542 describes the synthesis and biological evaluation of compounds having structure F. These compounds were evaluated as alternative substrates for avian liver farnesyl diphosphate and lemon peel cyclase.

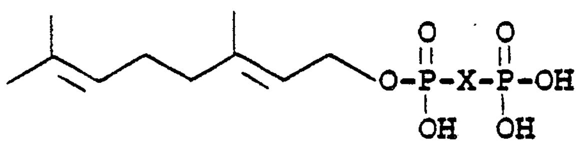

F X=$CH_2$, $CF_2$

McClard, R. W. and Poulter, C. D., et al., J.A.C.S. 1987, 109, 5544, reported that phosphinylphosphonates G and H were competitive inhibitors of the 1′-4-condensation between isopentenyl diphosphate and geranyl diphosphate catalyzed by avian liver farnesyl diphosphate synthetase. Phosphinylphosphonates G and H had Ki's of 19µM and 71µM, respectively. They also reported the speculative isolation of the farnesyl phosphinylphosphonate I, and the geranyl phosphinylphosphonate J from the enzymatic reaction of G with geranyl pyrophosphate or dimethylallyl pyrophosphate, respectively. The structures of I and J were tentatively assigned based on relative TLC mobilities. They hypothesized that I could be a potential inhibitor of squalene synthetase.

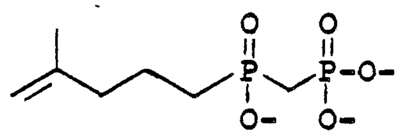

G

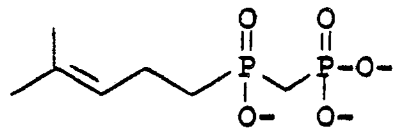

H

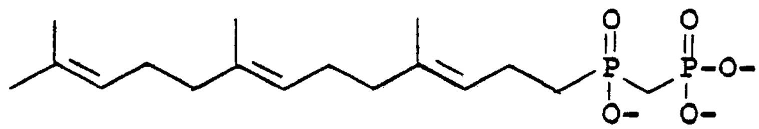

I

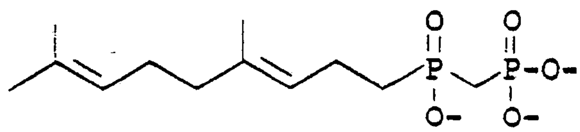

J

In accordance with the present invention, there is provided phosphorus-containing compounds which inhibit the enzyme squalene synthetase and thus are useful as hypocholesterolemic agents and have the following structure

$$\text{I.}\quad CH_3-\underset{CH_3}{C}=CH-CH_2-CH_2-\underset{CH_3}{C}=CH-Q-(CH_2)_n-X-\overset{O}{\underset{OR^1}{P}}-\overset{R^2}{\underset{R^3}{C}}-\overset{O}{\underset{OR^{1a}}{P}}-OR$$

wherein Q is

$$-(CH_2)_2-\underset{CH_3}{C}=CH-$$

or a bond;
n is 1, 2, 3 or 4;
X is -O-, -NH-, or $-NR^4-$;
R, $R^1$ and $R^{1a}$ may be the same or different and are H, lower alkyl, lower alkenyl or a metal ion;
$R^2$ and $R^3$ may be the same or different and are H or halogen;
$R^4$ is lower alkyl;
with the proviso that when X is O, n is 2, 3 or 4

Hereinafter the moiety

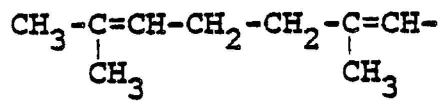

will be expressed as "Z" in the structural formulae set out below.

Thus, the following types of compounds are included within the scope of the present invention.

IA.

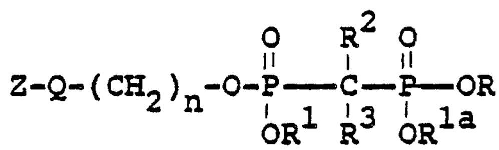

where n is 2, 3 or 4

IB.

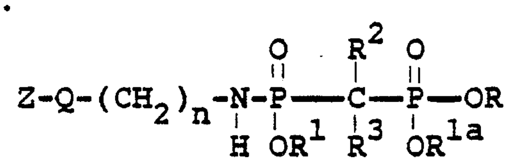

IC.

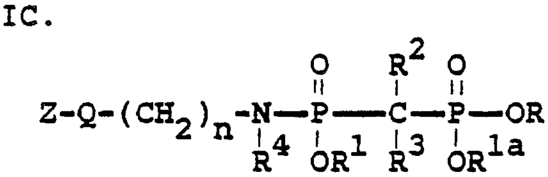

Unless otherwise indicated, the term "lower alkyl" or "alkyl" as employed herein alone or as part of another group includes both straight and branched chain hydrocarbons, containing 1 to 6 carbons in the normal chain, preferably 1 to 4 carbons, such as methyl, ethyl, propyl, isopropyl, butyl, t-butyl, isobutyl, pentyl, hexyl or isohexyl.

The term "lower alkenyl" as used herein by itself or as part of another group refers to straight or branched chain radicals of 3 to 12 carbons, preferably 3 to 6 carbons in the normal chain, which include one double bond in the normal chain, and which may include an aryl or alkyl substituent, such as 2-propenyl, 2-butenyl, 3-phenyl-2-propenyl, 2-pentenyl, 2-hexenyl, 2-heptenyl, 2-octenyl, 2-nonenyl, 2-decenyl, 2-undecenyl, 2-dodecenyl and the like.

The term "halogen" or "halo" as used herein refers to chlorine, bromine, fluorine, and iodine with chlorine or fluorine being preferred.

The term "metal ion" refers to alkali metal ions such as sodium, potassium or lithium and alkali earth metal ions such as magnesium.

Preferred are those compounds of formula I which have the following structure

ID. 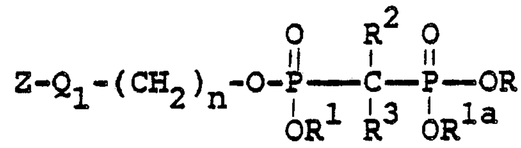

wherein $Q_1$ is

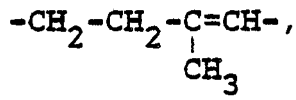

n is 2 or 3,
$R^2$ and $R^3$ are each H or each F; R, $R^1$ and $R^{1a}$ are H or metal ions
or

IE. 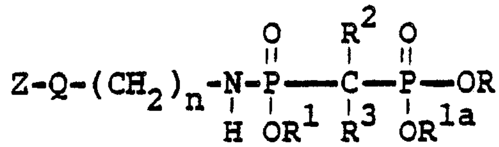

wherein Q is

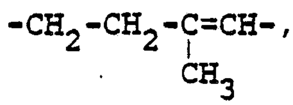

n is 1,
$R^2$ and $R^3$ are each H or each F; R, $R^1$ and $R^{1a}$ are alkyl, H or metal ions.

The compounds of formula I of the invention may be prepared according to the following reaction sequences and descriptions thereof.

## Preparation of Compounds of the Invention

A. Compounds of formula I of the invention wherein X is -O- and n is 2, 3 or 4 (that is, compound ID) may be prepared using the methylene bisphosphonate synthesis methodology developed by Poulter, C.D. et al., J. Org. Chem., 1986, 51, 4768, as outlined below:

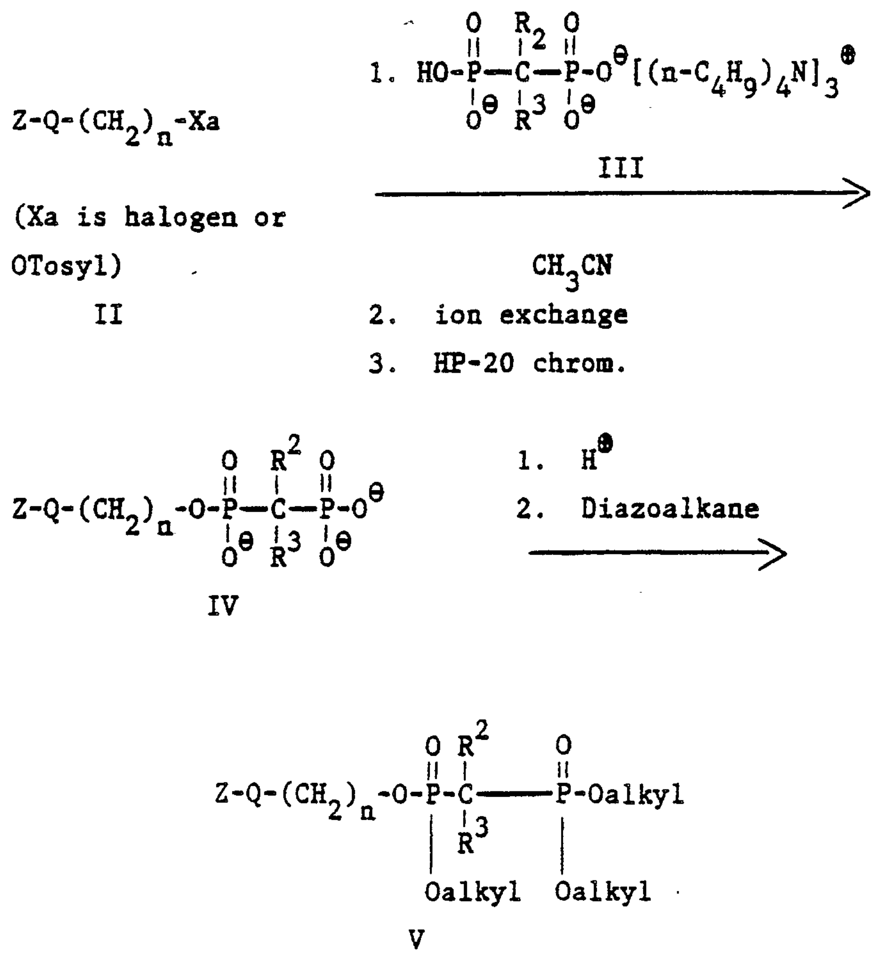


(Xa is halogen or OTosyl)

II

IV

V

B. Compounds of formula I wherein X is 0, and R is lower alkyl may be prepared according to the following reactions:

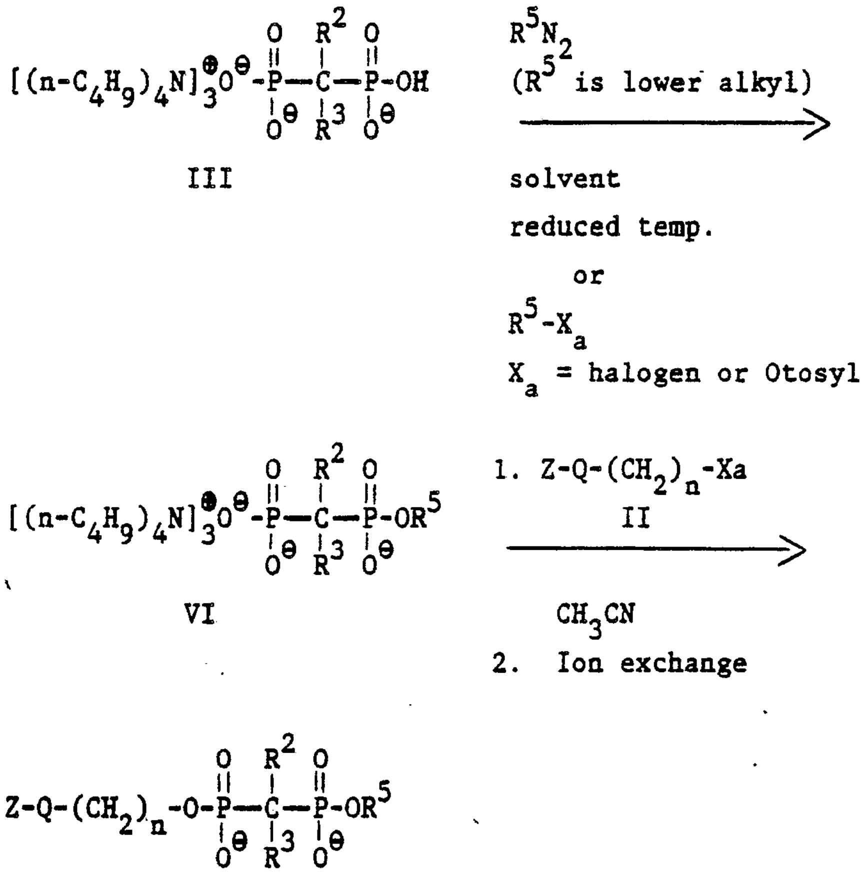


III

VI

VII

(wherein $R^5$ is lower alkyl)

B′. In an alternative method, compounds of formula I where X is O and R is lower alkyl may be prepared according to the following reaction:

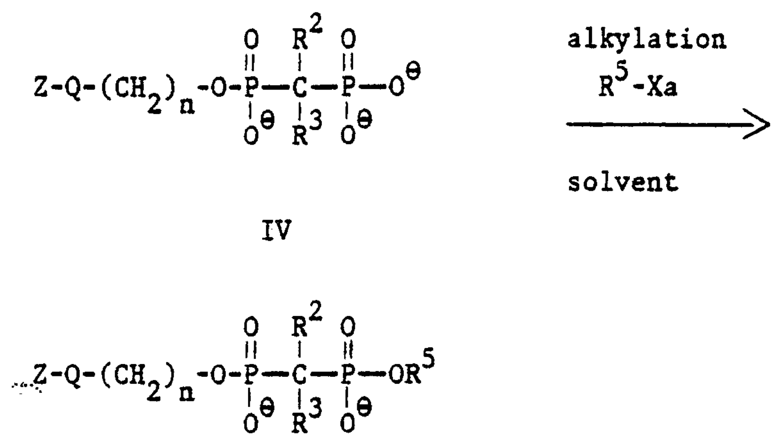


IV

VII

C. Triester compounds of formula I wherein X is -NH- or -NR⁴- may be prepared according to the following reaction sequence:

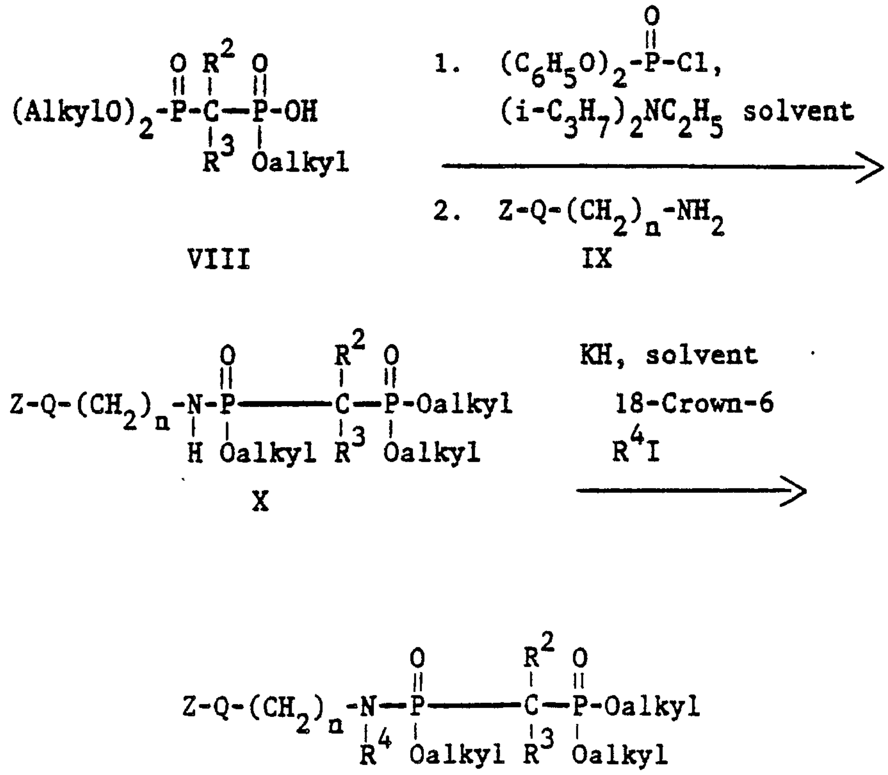


VIII IX

X

XI

D. Di- and tri-acid salts of formula I where X = -NH- and -NR⁴- may be prepared according to the following scheme:

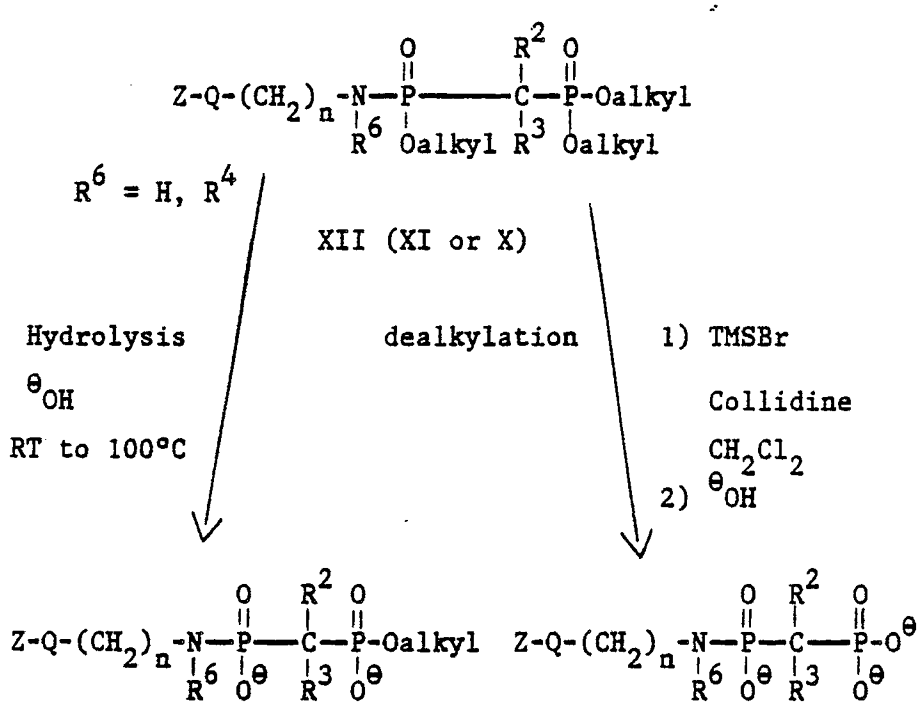


XIII XIV

The amine starting material

IX $Z\text{-}Q\text{-}(CH_2)_n\text{-}NH_2$

may be prepared by a Gabriel Synthesis according to the following reaction sequence:

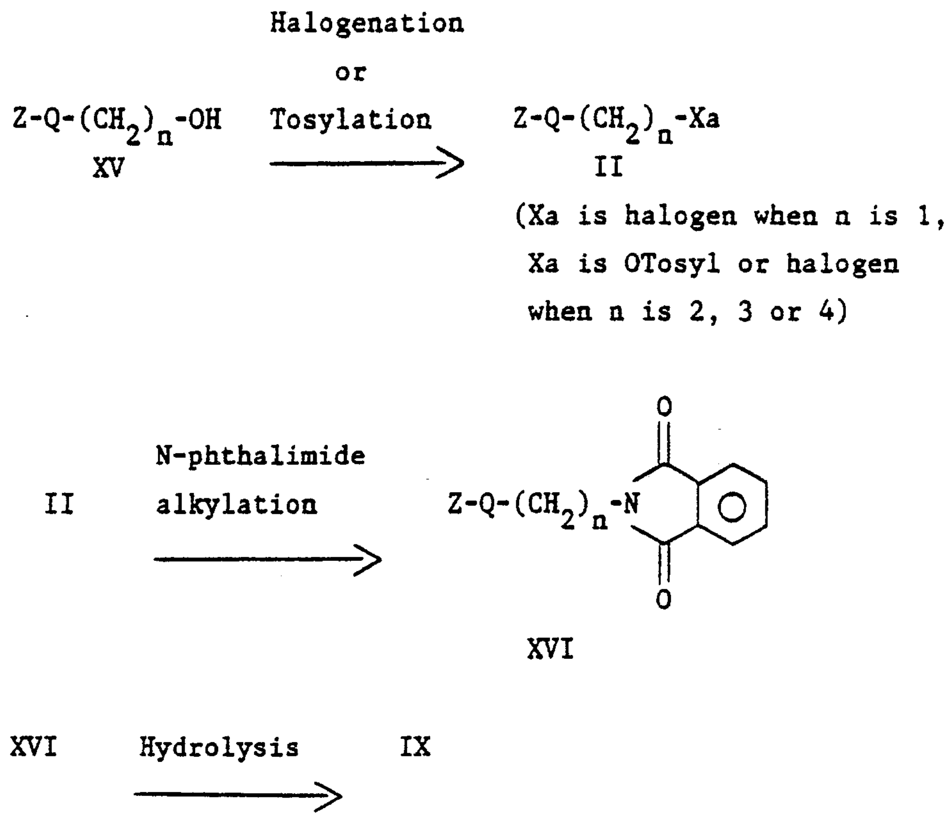


XV II

II XVI

XVI IX

As seen in the above reaction sequence A, compounds of formula V of the invention may be prepared by treating compound II

II $Z\text{-}Q\text{-}(CH_2)_{n'}\text{-}Xa$

(where Xa is Cl or Otosyl)

with dry tris(tetra-n-butyl)ammonium hydrogen methanediphosphate III

III

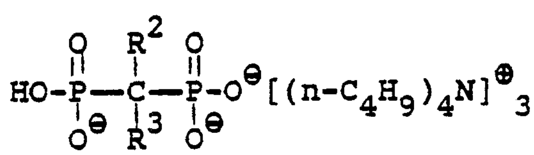

in an inert organic solvent such as dry acetonitrile ($CH_3CN$), nitromethane ($CH_3NO_2$) or methylene chloride ($CH_2Cl_2$) under an inert atmosphere such as argon, employing a molar ratio of III:II of within the range of from about 1:1 to about 5:1 and preferably about 3:1. The solvent is removed and the residue is run through an ion exchange column to form compound IV as an alkali metal or ammonium salt.

Compound IV is converted to the free acid by typical acid-base extraction, and the free triacid may be treated with a diazoalkane to form the ester V. The latter reaction is carried out employing a molar ratio of diazoalkane:IV of within the range of from about 3:1 to about >10:1 and preferably from about 3:1 to about 4:1.

In reaction sequence B, compounds of formula I wherein X is O and R is lower alkyl may be prepared by treating III with a diazoalkane ($R_1N_2$) in the presence of a suitable solvent such as ethyl ether or benzene, at a reduced temperature of within the range of from about 0 to about -10° C employing a molar ratio of diazoalkane:III of within the range of from about 1:1 to about 1.5:1. The resulting reaction product VI is reacted with tosyl compound II in the presence of an organic solvent such as acetonitrile, $CH_3NO_2$ or $CH_2Cl_2$ employing a molar ratio of VI:II of within the range of from about 1:1 to about 5:1 and preferably

from about 1:1 to about 3:1, to form the monoester of the invention VII.

In carrying out the alternative method for preparing compounds of formula I where X is O and R is lower alkyl, the alkylation reaction B′ is carried out employing a molar ratio of $R^5$-Xa:IV of within the range of from about 1:1 to about 1.5:1. The reaction is carried out in the presence of an inert organic solvent such as acetonitrile or dimethylformamide at a reduced temperature of from about 0 to about 100° C.

Referring to reaction sequence C, wherein compounds of formula I and intermediates where X is -NH- and -$NR^4$- are prepared, a solution of trialkylmethylene diphosphonate VIII and diisopropylethylamine in an organic solvent such as methylene chloride, under an inert atmosphere such as argon, is treated with diphenylchlorophosphate. The resulting solution is reacted with amine X employing a molar ratio of VIII:IX of within the range of from about 1:1 to about 1.2:1 to form the trialkyl compound of the invention XI.

Compounds of formula IC where X is -$NR^4$- may be prepared by treating X with KH, 18-crown-6 and $R^4$I at a temperature of about 0° C to about 65° C employing a molar ratio of $R^4$I TO X of within the range of from about 2:1 to about 3:1.

Referring now to reaction sequence D, compounds XII, where $R^6$ = H or $R^4$, can be converted to the corresponding monoester XIII by treatment with excess hydroxide between room temperature and about 100° C. Compounds XII can be dealkylated to triacid salts XIV by reaction with bromotrimethyl silane (TMSBr) (from about 3 to about 6 equiv.) and 2,4,6-collidine (from about 1 to about 4 equiv) in dichloromethane between about 0° C and room temperature, followed by hydrolysis with hydroxide.

## Starting Materials

The various amine or alcohol starting materials are either known or are prepared by procedures known in the art, such as described in the working Examples. For example, amine starting material IX may be prepared by a Gabriel synthesis wherein alcohol XV is subjected to halogenation or tosylation to form compound II which is treated with N-phthalimide to form compound XVI which may be hydrolyzed to IX.

The compounds of the present invention are inhibitors of squalene synthetase and thus are useful in inhibiting cholesterol biosynthesis and treating hypercholesterolemia and atherosclerosis. Inhibition of squalene synthetase may be measured by the following procedure.

Rat liver microsomal squalene synthetase activity is measured using farnesyl pyrophosphate as substrate and quantitating squalene synthesis using gas chromatographic analysis. The assay was developed by modifying conditions originally described by Agnew (Methods in Enzymology 110:357, 1985).

## Preparation of Rat Liver Microsomes:

Livers are dissected from 2 or 3 decapitated Sprague Dawley rats and are quickly transferred to ice cold buffer (potassium phosphate, 0.05 M, (pH 7.4); $MgCl_2$, 0.004 M; EDTA, 0.001 M; and 2-mercaptoethanol 0.01 M) and rinsed thoroughly. The livers are minced in cold buffer (2.0 ml/g) and homogenized using a Potter-Elvejhem homogenizer. The homogenate is centrifuged at 5,000 x g, 10 minutes (4° C), and the supernatant poured through 2 layers of cheese cloth. The supernatant is then centrifuged at 15,000 x g for 15 minutes (4°). Again the supernatant is filtered through 2 layers of cheese cloth, and centrifuged a third time at 100,000 x g for 1.0 hour at 4° C. Following centrifugation the microsomal pellet is resuspended in a volume of buffer equivalent to 1/5 the volume of the original homogenate, and homogenized in a ground glass homogenizer. Aliquotted microsomes are frozen at -80° C, and retain activity for at least two months.

## Enzyme Assay:

Reaction Mixtures are prepared in 50 ml round bottom pyrex glass tubes with tight-fitting, teflon-lined, screw caps. Tubes are cooled to 4° C, and the following components are added in sequence:

| | | |
|---|---|---|
| 1. | Potassium phosphate buffer 0.275 M, pH 7.4) | 0.36 ml |
| 2. | KF (55 mM) | 0.36 ml |
| 3. | NADPH (5.0 mM, freshly prepared) | 0.36 ml |
| 4. | $H_2O$ (or $H_2O$ + test compound) | 0.16 ml |
| 5. | $MgCl_2$ (27.5 mM) | 0.36 ml |
| 6. | Microsomal Enzyme (0.48 mg microsomal protein in homogenization buffer) (15 µl prep. 4/23/86 | 0.20 ml |
| | | 1.8 ml |

This mixture is equilibrated under $N_2$ at 4° C for 5-15 minutes. Reaction mixtures are then warmed to 30° C, and the enzyme reaction initiated by adding 0.2 ml of farnesyl pyrophosphate (219 µM) prepared in $H_2O$. Each tube is again overlayered with $N_2$, and incubated at 30° C for 60 minutes. The reaction is stopped by the addition of 1.0 ml KOH (40%). Ethanol (95%) (spectral grade) (1.0 ml) is added to each tube. Docosane (5 nmoles in hexane) is added to each tube as an internal standard. The mixture is saponified at 65° C for 30 minutes. The tubes are cooled to room temperature and extracted twice with 10.0 ml spectral grade hexane.

The upper organic phase fractions are pooled in glass 20.0 ml scintillation vials and reduced in volume to ≈1.0 ml under a stream of $N_2$. The sample is then transferred to acid-washed, conical bottom, glass (1.0 ml) microvials, and brought to dryness under $N_2$. The residue is resuspended in 50 µl hexane (spectral grade), and these samples are spun at 1000 rpm at room temperature for 10 minutes. Following centrifugation approximately 40 µl of supernatant is transferred to 100 µl acid-washed microvials with septa/crimp-top caps (compatible with the Hewlett-Packard GC auto injector).

Gas Chromatography:

Two µL of each sample is injected onto a fused silica megabore DB-17 column (15 M x 0.525 mm) (J&W Scientific) using a splitless mode of injection. Gas flow rates are listed below:

| | | |
|---|---|---|
| Make up gas (helium) | | 20 ml/min. |
| Air | | 400 ml/min. |
| Hydrogen | | 30 ml/min. |
| Carrier (helium) | | 15 ml/min |
| Septum purge vent | | 5 ml/min. |
| | (Septum purge off 0.00 min., on at 0.5 min.) | |

The injector temperature is 200° C, and the FID detector temperature is set at 270° C. Oven temperature is programmed through a two ramp sequence as follows:

Oven:

Initial temperature 180° C, initial time 10 minutes
Ramp one: 20° C/minute
Second temperature 250° C, second time 10 minutes
Ramp two: 20° C/minute
Third temperature 260° C, third time 10 minutes
(Equilibration time 1.0 minute)

Using this gas chromatographic system, docosane (internal standard) has a retention time of 3.6-3.7 minutes, and squalene has a retention time of 14.7-14.9 minutes. The amount of squalene in each reaction mixture is determined by obtaining the areas under the squalene and docosane peaks and using the following formula to calculate the amount of squalene (nmoles) in the total reaction mixture.

$$\text{Squalene (nmoles/reaction mixture)} = 5.0 \text{ (nmoles docasane internal standard)} \times \frac{\text{Squalene Peak Area}}{\text{Docasane Peak Area}} \times RR^{*}$$

*RR = Response Ratio [Docasane/Squalene]

*RR = 0.56

Compounds Testing:

Compounds are dissolved in $H_2O$ and added to reaction mixtures prior to addition of farnesyl pyrophosphate substrate. All reaction mixtures are run in duplicate, at several concentrations. Additionally, all compound $I_{50}$ values are derived from composite dose response data with the 95% confidence interval indicated.

A further aspect of the present invention is a pharmaceutical composition consisting of at least one of the compounds of Formula I in association with a pharmaceutical vehicle or diluent. The pharmaceutical compostion can be formulated employing conventional solid or liquid vehicles or diluents and pharmaceutical additives of a type appropriate to the mode of desired administration. The compounds can be administered to mammalian species including humans, monkeys, dogs, etc. by an oral route, for example, in the form of tablets, capsules, granules or powders, or they can be administered by a parenteral route in the form of injectable preparations. The dose for adults is preferably between 200 and 2,000 mg per day, which can be administered in a single dose or in the form of individual doses from 1-4 times per day.

A typical capsule for oral administration contains active ingredient (250 mg), lactose (75 mg) and magnesium stearate (15 mg). The mixture is passed through a 60 mesh sieve and packed into a No. 1 gelatin capsule.

A typical injectible preparation is produced by asceptically placing 250 mg of sterile active ingredient into a vial, asceptically freeze-drying and sealing. For use, the contents of the vial are mixed with 2 ml of physiological saline, to produce an injectible preparation.

The following Examples represent preferred embodiments of the present invention. Unless otherwise indicated, all temperatures are expressed in degrees Centigrade. Purification of final targets was often achieved by chromatography on CHP20P gel (referred to herein as HP-20), a highly porous styrene-divinyl benzene copolymer available from Mitsubishi Chemical Industries. $^{31}P$ NMR spectra were accumulated in the $^{1}H$ decoupled mode, employing 85% $H_3PO_4$ ($\delta$ = 0 ppm) as the external reference.

## Example 1

[[Methoxy[(3,7,11-trimethyl-2,6,10-dodecatrienyl)amino]phosphinyl]methyl]phosphonic acid, dimethyl ester

### A. Trimethyl methylene diphosphonate

Tetramethylmethylene diphosphonate (Lancaster Synthesis #5847) (2.50 g, 10.8 mmol) in a mixture of 8.65 ml of methanol and 8.65 ml (8.65 mmol, 0.8 eq.) of 1M NaOH was stirred at room temperature under argon for 24 hours. After removing the solvent under reduced pressure, the remaining starting material was removed by extraction with five 15 ml portions of $CH_2Cl_2$. The aqueous phase was loaded onto a 90 ml (10 eq.) AG-50W-X4 ion-exchange columr in the acid form and eluted with water. Fractions containing the desired product were combined, and water was removed under reduced pressure. The residue was

dissolved in 75 ml of $CH_2Cl_2$, dried over $Na_2SO_4$ overnight and evaporated to provide 1.63 g (69%) of title compound as a clear, viscous oil.
TLC: Silica gel (6:3:1 n-$C_3H_7OH$:con.$NH_3$:$H_2O$) Rf = 0.48.
$^1$H NMR ($CDCl_3$) (270 MHz)
δ 11.55 (br s, 1H)
3.82 (d, 6H, J = 11.5 Hz)
3.80 (d, 3H, J = 11.5 Hz)
2.56 (t, 2H, J = 21 Hz) ppm.
Mass Spec (CI-$NH_3$, + ions) m/e 236 (M + H + $NH_3$), 219 (M + H).

B. Farnesyl amine

(1) 2-(3,7,11-Trimethyl-2,6,10-dodecatrienyl)-1H-isoindole-1,3(2H)-dione

A solution of 2.00 g (9.0 mmol) of trans, trans-farnesol 98% (obtained from Aldrich (27,754-1) and used without further purification) in 20 ml of dry ethyl ether at 0°C under argon in the dark was treated dropwise with a solution of 735 µl (4.0 mmol, 0.45 eq.) of $PBr_3$ in 4 ml of dry ethyl ether. The resultant mixture was stirred at 0°C for one hour, then quenched with $H_2O$ and separated. The organic phase was washed with 15 ml of $NaHCO_3$, 15 ml of $H_2O$, and 15 ml of brine, dried over $MgSO_4$ and evaporated to provide 2.47 g of crude farnesyl bromide as a clear oil.
TLC:Silica gel (2:8 ethyl acetate:Hexane) Rf = 0.63

A solution of 2.47 g of farnesyl bromide in 20 ml of dry dimethyformamide (DMF) at room temperature under argon was treated with 1.83 g (9.9 mmol, 1.1 eq.) of potassium phthalimide and stirred for 3 hours at room temperature. The solvent was removed under reduced pressure, the residue was triturated with 150 ml of ethyl ether, and the precipitate was filtered off. The ethereal solution was washed with 50 ml of $H_2O$ and 50 ml of brine, dried over $MgSO_4$ and evaporated to yield 2.96 g of crude title compound as a milky oil. Purification by flash chromatography on 300 g of Merck 9385 silica, eluted with 7:93 ethyl acetate:petroleum ether afforded 2.56 g (81%) of the desired product as a colorless oil.
TLC:Silica gel (2:8 ethyl acetate:Hexane) Rf = 0.37 IR(neat) 2967, 2920, 2856, 1772, 1716, 1468, 1432, 1394, 1366, 1325, 1112, 1088, 1073, 947, 721, 531 $cm^{-1}$.
$^1$H NMR ($CDCl_3$) (270 MHz)
δ 7.82 (dd, 2H, J = 3.0, 5.5 Hz)
7.68 (dd, 2H, J = 3.0, 5.5 Hz)
5.27 (t, 1H, J = 7.0 Hz)
5.05 (d, 2H, J = 7.0 Hz)
4.27 (d, 2H, J = 7.0 Hz)
1.9-2.1 (m, 8H)
1.83 (s, 3H)
1.66 (s, 3H)
1.56 (s, 6H) ppm.
Mass Spec (CI-$CH_4$/$N_2O$ + ions) m/e 392 (M + $C_3H_5$), 380 (M + $C_2H_5$), 352 (M + H), 296, 284, 270, 228, 216.

(2) Farnesyl amine

A solution 2.50 g (7.1 mmol) of Part (1) compound in 15 ml of absolute ethanol at room temperature under argon was treated with 1.9 ml (35.57 mmol, 5.0 eq.) of methyl hydrazine and stirred for 2 hours at room temperature and 4 hours at reflux. After cooling and the addition of 7.1 ml (7.1 mmol, 1.0 eq.) of 1M NaOH, the ethanol was removed under reduced pressure. The residue was extracted with 350 ml of ethyl ether and the ether layer washed with 100 ml of 1M NaOH, 50 ml of $H_2O$ and 50 ml of brine, dried ($MgSO_4$) and evaporated to obtain 1.45 g of crude product. Purification by Kugelrohr distillation at 120°C/0.005 mm provided 1.405 g (89%) of title compound as a colorless oil.
TLC:Silica gel (8:1:1 n-propanol:con.$NH_3$:$H_2O$) Rf = 0.64
IR(neat) 3291, 2967, 2923, 2856, 1574, 1483, 1453, 1383, 1378, 1347, 1288, 819, 777 $cm^{-1}$
$^1$H NMR ($CDCl_3$) (270 MHz)
δ 5.26 (t, 1H, J = 7.0 Hz)

5.10 (br, 2H)
3.27 (d, 2H, J = 7.0 Hz)
1.9-2.1 (m, 8H)
1.68 (s, 3H)
1.63 (s, 3H)
1.60 (s, 6H)
1.20 (br s, 2H) ppm.
Mass Spec (CI-$CH_4$/$N_2O$, + ions) m/e 443 (2M + H), 222 (M + H), 205, 137.

C. [[Methoxy[(3,7,11-trimethyl-2,6,10-dodecatrienyl)amino]phosphinyl]methyl]phosphonic acid, dimethyl ester

To a solution of 620 mg (2.84 mmol, 1.1 eq.) of Part A compound and 495 µL (2.84 mmol, 1.1 eq.) of diisopropylethylamine in 5 ml of dry $CH_2Cl_2$ at room temperature under argon was added 590 µL (2.84 mmol, 1.1 eq.) of diphenylchlorophosphate. The resulting solution was stirred for 2 hours, then cooled to 0° C. A mixture of 525 mg (2.37 mmol) of Part B farnesyl amine and 515 µL (2.96 mmol, 1.25 eq.) of diisopropylethylamine in 5 ml of dry $CH_2Cl_2$ was added dropwise and the resulting solution was stirred for 4 hours. The reaction mixture was diluted with 50 ml of ethyl ether and washed with 10 ml of $NaHCO_3$ and 10 ml of brine, dried over $MgSO_4$ and evaporated to yield 1.13 g of crude product. Purification by flash chromatography on 120 g of Merck 9385 silica, eluted with 4:96 $CH_3OH$:$CH_2Cl_2$ provided 479.6 mg (48%) of pure title product and 89.7 mg (9%) of slightly impure title product.
TLC:Silica gel (5:95 $CH_3OH$:$CH_2Cl_2$) Rf = 0.21.
IR($CCl_4$) 2953, 2926, 2853, 1254, 1184, 1038, 849, 819 $cm^{-1}$.
$^1H$ NMR($CDCl_3$) (270 MHz)
δ 5.25 (t, 1H, J = 7 Hz)
5.09 (m, 2H)
3.79 (d, 3H, J = 11 Hz)
3.77 (d, 3H, J = 11 Hz)
3.70 (d, 3H, J = 11 Hz)
3.62 (m, 2H)
3.25 (m, 1H)
2.45 (dt, 1H, J = 15.5, 20.5 Hz)
2.38 (dt, 1H, J = 15.5, 20.5 Hz)
1.9-2.1 (m, 8H)
1.67 (s, 6H)
1.60 (s, 6H) ppm.
Mass Spec (CI-$CH_4$/$N_2O$, + ions) m/e 843 (2M + H), 462 (m + $C_3H_5$), 450 (M + $C_2H_5$), 422 (m + H), 218.
Also isolated as a by-product was 291.2 mg (27%) of the following compound

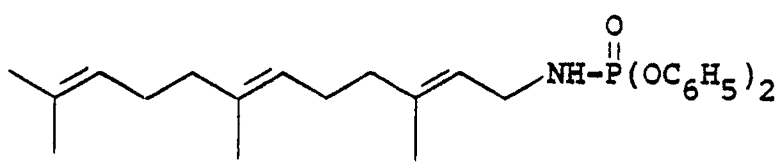

TLC:Silica gel (5:95 $CH_3OH$:$CH_2Cl_2$) Rf = 0.84.

## Example 2

[[Hydroxy[(3,7,11-trimethyl-2,6,10-dodecatrienyl)amino]phosphinyl]methyl]phosphonic acid, methyl ester, disodium salt

A mixture of 450.2 mg (1.07 mmol) of Example 1 compound in 5.35 ml (5.35 mmol, 5 eq.) of 1M NaOH was heated at 60-65 °C under nitrogen for 16 hours. The aqueous solution (pH 13) was loaded onto a 2.5 cm diameter x 18 cm height column of HP-20 packed with water. The column was eluted with a gradient created by the gradual addition of 350 ml of acetonitrile to 350 ml of 2:98 acetonitrile:water. Approximately 10 ml fractions were collected every two minutes. Fraction 25 contained pure title product and was evaporated, lyophilized and pump-dried overnight to obtain 104.1 mg (22%) of pure title product. (Fractions 26-27 provided 230.4 mg (49%) of title product, containing a minor impurity cospotting on TLC with Example 1 Part B farnesyl amine). A 1.0% solution of title product gave pH 8.55.

TLC:Silica gel (7:2:1 n-$C_3H_7OH$:con.$NH_3$:$H_2O$) Rf = 0.43

IR(KBr) 3378, 2967, 2924, 2853, 1444, 1419, 1405, 1380, 1217, 1153, 1109, 1061, 789, 753, 510, 497, 483, 468 $cm^{-1}$.

$^1H$ NMR ($D_2O$) (400 MHz)
δ 5.26 (t, 1H, J = 6.5 Hz)
5.15 (t, 1H, J = 6.5 Hz)
5.12 (t, 1H, J = 6.0 Hz)
3.50 (d, 3H, J = 10.6 Hz)
3.38 (t, 2H, J = 6.5 Hz)
1.9-2.1 (m, 10H)
1.63 (s, 3H)
1.61 (s, 3H)
1.56 (s, 6H) ppm.

$^{31}P$-NMR ($D_2O$) (36 MHz)
δ 20.0
19.2 ppm.

Mass Spec (FAB, + ions) m/e 460 (M + Na), 438 (M + H), 411, 217.

| Anal Calcd for $C_{17}H_{31}NO_5P_2 \cdot Na_2$ (MW 437.370)*: | |
|---|---|
| | C, 46.69; H, 7.14; N, 3.20; P, 14.16 |
| Found: | C, 46.42; H, 7.51; N, 3.13; P, 14.26 |

*Sample was dried to constant weight at 50 °C.

## Example 3

**(E,E)-[[Hydroxy[(4,8,12-trimethyl-3,7,11-tridecatrienyl)oxy]phosphinyl]methyl]phosphonic acid, tripotassium salt**

A. 4,8,12-Trimethyl-3,7,11-tridecatrien 1-ol, (4-methylphenyl)sulfonate

(1) 3,7,11-Trimethyl-2,6,10-dodecatrien-aldehyde (E,E-Farnesal)

A solution of oxalyl chloride (4.68 g, 0.037 mmol) in dry $CH_2Cl_2$ under argon atmosphere was cooled to -65 °C. A solution of dimethyl sulfoxide (DMSO) (5.33 ml) in $CH_2Cl_2$ (17 ml) was added rapidly, dropwise, to the cooled oxalyl chloride solution. After stirring for 7 minutes at -65 °C, a 10 ml $CH_2Cl_2$ solution of farnesol (7.0 g, 0.032 ml; obtained from Aldrich, 98% trans-trans and used without further purification) was added over 10 minutes to the reaction solution at -65 °C: a precipitate formed upon the addition of approximately half of the farnesol solution. After the addition of the farnesol solution was completed, the reaction was stirred at -65 °C for 25 minutes, and then 22.4 ml of triethyl amine was added over 10 minutes. After stirring for an additional 15 minutes at -65 °C, the reaction was warmed to room temperature, and then diluted with

water (~200 ml). The resulting aqueous layer was extracted several times with $CH_2Cl_2$. The combined organic layers were washed once with saturated aqueous NaCl solution once with 1% HCl, once with 5% $Na_2CO_3$ solution and once with saturated aqueous NaCl solution. The resulting organic layer was dried over $MgSO_4$ to give 7.05 g (100%) of a clear oil after filtration and solvent removal: 10:1 2E:2Z double bond.
TLC:Silica gel: (20% ethyl acetate/hexane). Rf = 0.34.
$^1$H NMR ($CDCl_3$) (270 MHz)
δ 9.98 (d, 1H, J = 7 Hz, CHO)
5.88 (broad d, 1H, J = 7 Hz, CHCHO)
5.08 (m, 2H, $CH_2CHCCH_3$, $CH_2CHC(CH_3)_2$)
2.22 (m, 4H)
2.17 (s, 3H, $CH_3CCHCHO$)
2.02 (m, 4H)
1.66 (s, 3H, $CH_2CCH_3CH$)
1.60 (s, 6H, $(CH_3)_2CCH_2$)
$^{13}$C-NMR ($CDCl_3$) (67.8 MHz)
δ 191.0, 163.6, 136.5, 131.3, 127.4, 124.0, 122.4, 40.5, 39.6, 26.6, 25.6, 17.6, 17.5, 15.9

(2) 4,8,12-Trimethyl-1,3,7,11-tridecatetraene

A suspension of methyltriphenylphosphonium iodide (8.07 g, 0.02 mole) in 61 ml of dry tetrahydrofuran (THF), under argon atmosphere was cooled to 0 °C. To this suspension at 0 °C was added phenyllithium (9.0 ml solution:2.0 M in ethyl ether/hexane 30:70) over 10 minutes. After the addition was complete, the reaction mixture containing excess phosphonium salt was warmed to room temperature and stirred for 40 minutes. The reaction mixture was then recooled to 0 °C, and a 10 ml THF solution of the Part (1) aldehyde (4.0 g, 0.018 mol) was added over 12 minutes. After stirring for 10 minutes at 0 °C, the reaction was warmed to room temperature. The reaction was quenched with $CH_3OH$ after 2 hours at room temperature. The THF was removed from the reaction mixture to give a slurry which was triturated with petroleum ether, and subsequently, filtered through a Celite pad in a sintered glass funnel. The solids were then boiled in petroleum ether and refiltered as above. The resulting yellow oil was passed through 50 g of Florisil (100-200 mesh) which had been pre-equilibrated with petroleum ether. The Florisil column was eluted with ~400 ml of petroleum ether providing the title tetraene (3.36 g, 86%) as a clear oil after solvent removal.
TLC:Silica gel (20% ethyl acetate/hexane) Rf = 0.68
$^1$H NMR ($CDCl_3$) (270 MHz)
δ 6.56 (ddd, 1H, J = 17, 12, 6 Hz, $CCHCHCH_2$)
5.85 (d, 1H, J = 12 Hz, $CCHCHCH_2$)
5.10 (m, 2H, $CCHCH_2$)
5.02 (m, 2H, $CHCH_2$)
2.05 (m, 8H, $CHCH_2CH_2C$)
1.75 (s, 3H, $CH_2CH_3CCH$)
1.67 (s, 3H, $CH_2CH_3CCH$)
1.60 (s, 6H, $(CH_3)_2C$)
$^{13}$C-NMR ($CDCl_3$) (67.8 MHz)
δ 139.3, 135.3, 133.4, 131.2, 125.5, 124.3, 123.9, 114.5, 39.9, 39.7, 26.8, 26.4, 25.6, 17.7, 16.6, 15.9

(3) 4,8,12-Trimethyl-3,7,11-tridecatrien-1-ol

Neat 2-methyl-2-butene (2.25 g, 0.032 mol) was added to a 1.0 M $BH_3$-THF solution (16.9 ml) at -50 °C and under argon. After the addition was complete, the reaction was stirred for 2 hours at 0 °C. The resulting disiamylborane solution was transferred via cannula to a flask containing a 17 ml THF solution of Part A(2) tetraene (3.36 g, 0.015 mol) under argon atmosphere and cooled to 0 °C. The transfer procedure was accomplished over 1 hour. After the transfer was complete, the reaction was allowed to gradually warm to room temperature, and then it was stirred overnight at room temperature. After stirring overnight, the reaction mixture was cooled to 0 °C, and 5.1 ml of 3N NaOH was added rapidly. After stirring for 10 minutes, the reaction mixture was cooled in an ice-salt bath and 5.1 ml of 30% $H_2O_2$ was added dropwise. Subsequently, the reaction was warmed to room temperature and stirred for 4 hours after which it was diluted with $H_2O$, and the resulting aqueous layer was extracted several times with ethyl ether. The

combined organic layers were dried over $MgSO_4$. Purification by flash chromatography eluting with 20% ethyl acetate/hexane provided the title alcohol (2.62 g, 74%) as a clear oil.

TLC:Silica gel (20% ethyl acetate/hexane) Rf = 0.23

IR (Film) 3340 (br), 2965, 2920, 1665 (w), 1440, 1380, 1100 (w), 1050 $cm^{-1}$.

$^1H$ NMR ($CDCl_3$) (270 MHz)

δ 5.10 (m, 3H, $CH_3\underline{C}HC$)

3.61 (t, 2H, J = 6 Hz, $HOC\underline{H}_2CH_2$)

2.29 (q, 2H, J = 6 Hz, $CHC\underline{H}_2CH_2OH$)

2.03 (m, 8H $CHCH_2CH_2C$)

1.67 (s, 3H, $CC\underline{H}_3CHCH_2CH_2OH$)

1.65 (s, 3H, $CH_2CCH_3CH$)

1.60 (s, 6H, $CHC(C\underline{H}_3)_2$)

$^{13}C$-NMR ($CDCl_3$) (67.8 MHz)

δ 138.8, 135.2, 131.2, 124.3, 123.9, 119.9, 62.4, 39.8, 39.7, 31.5, 26.7, 26.5, 25.6, 17.6, 16.1, 15.9

(4) 4,8,12-Trimethyl-3,7,11-tridecatrien-1-ol, (4-methylphenyl)sulfonate

A solution of Part (3) homofarnesol (0.307 g, 1.3 mmole) in 15 ml of dry $CH_2Cl_2$ was stirred at room temperature and under argon atmosphere with pyridine (0.52 g, 6.5 mmol) and dimethyl amino pyridine (DMAP) (0.032 g, 0.26 mmol). At room temperature, p-toluenesulfonyl chloride (TsCl) (0.55 g, 2.8 mmol) was added to the reaction mixture. After stirring for 20 hours, an additional 0.2 g of TsCl was added. After stirring for 20 hours, an additional 0.2 g of TsCl was added. After stirring for 32 hours at room temperature, the reaction was quenched with saturated aqueous $NaHCO_3$ solution and stirred vigorously for 45 minutes. The reaction mixture was then diluted with $H_2O$, and the aqueous layer was extracted several times with $CH_2Cl_2$. The combined $CH_2Cl_2$ extracts were washed once with 5% aqueous $CuSO_4$ solution and once with $H_2O$. The resulting organic extract was dried over $MgSO_4$. Filtration and solvent removal gave 0.50 g of a yellow oil. Purification by flash chromatography (silica gel; 15% ethyl acetate/hexane) provided 0.45 g (89%) of title tosylate as a clear oil.

TLC:Silica gel (20% ethyl acetate /hexane) Rf = 0.39

IR (film) 2965, 2920, 2860, 1600, 1445, 1360, 1190, 1175, 1100, 960, 910, 815 $cm^{-1}$.

$^1H$-NMR ($CDCl_3$) (270 MHz)

δ 7.78 (d, 2H, J = 7.0 Hz, Ar-H)

7.33 (d, 2H, J = 7.0 Hz, Ar-H)

5.07 (m, 2H $CC\underline{H}CH_2$)

4.98 (m, 1H, $CC\underline{H}CH_2CH_2OTs$)

3.98 (t, 2H, J = 7 Hz, $C_2C\underline{H}_2OTs$)

2.45 (s, 3H, Ar-$CH_3$)

2.35 (m, 2H, $CHC\underline{H}_2CH_2OTs$)

2.00 (m, 8H, $CHC\underline{H}_2C\underline{H}_2C$)

1.70 (s, 3H, $CH_3$)

1.60 (s, 3H, $C\underline{H}_3$)

1.58 (s, 3H, $C\underline{H}_3$)

1.55 (s, 3H, $C\underline{H}_3$)

$^{13}C$-NMR ($CDCl_3$): (67.8 MHz)

δ 144.5, 139.3, 135.2, 133.3, 131.2, 129.7, 127.8, 124.3, 123.8, 117.4, 69.9, 39.6, 39.5, 27.8, 26.7, 26.4, 25.6, 21.5, 17.6, 16.1, 15.9

B. (E,E)-[[Hydroxy[(4,8,12-trimethyl-3,7,11-tridecatrienyl)oxy]phosphinyl]methyl]phosphonic acid, tripotassium salt

Tris (tetra-n-butyl)ammonium hydrogen methanediphosphate (1.94 g, 2.16 mmol) (prepared by titrating to pH 10.0 a 20 ml deionized $H_2O$ solution of methanediphosphonic acid (1.0 g, 5.68 mmol, Fluka) with 40% w/w aqueous $(n\text{-}C_4H_9)_4$ NOH and lyophilization of the resulting aqueous solution) was dissolved in 2.0 ml of dry $CH_3CN$ followed by $CH_3CN$ removal via rotary evaporation. Additional $CH_3CN$ was added and removed via rotary evaporation to facilitate water removal. To the resulting residue under argon atmosphere was added 2.0 ml of dry $CH_3CN$ followed by the addition of a 2.0 ml $CH_3CN$ solution of Part A tosylate

(0.243 g, 0.63 mmol). After stirring for 2 hours at room temperature, the $CH_3CN$ was removed to give a clear residue which was dissolved in a 1:49 i-$C_3H_7OH$/0.025 M $K_2CO_3$ buffer solution and eluted through an ion exchange column (AG50W-8x, $K^+$-form 100-200 mesh, 50 ml of resin in a 20 mm diameter column) with the buffer solution. A milky white eluent eluted from the column. After lyophilization of the eluent, the lyophilate was resubjected to the ion exchange chromatography as above (eluted with 1:49 i-$C_3H_7OH/H_2O$). The resulting milky white eluent was lyophilized, and the lyophilate was purified by HP-20 chromatography (2.5 cm x 20 column of resin). Elution with a $H_2O$-acetonitrile gradient provided 0.14 g (44%) of title product, after lyophilization.

TLC:Silica gel Rf=0.43 (6:3:1 n-$C_3H_7OH$:Con $NH_3$:$H_2O$)
IR(KBr) 2966, 2923, 2857, 1666, 1447, 1382, 1211, 1087, 890, 800, 733 $cm^{-1}$.
$^1$H-NMR ($D_2O$) (400 MHz)
δ 5.19 (m, 3H, CCHCH₂)
3.82 (m, 2H, $CH_2\underline{CH_2}OPP$)
2.34 (m, 2H, $CH\underline{CH_2}CH_2OPP$)
2.05 (m, 10H, $CH\underline{CH_2}\underline{CH_2}C$)
1.67 (s, 3H, $C\underline{CH_3}$)
1.65 (s, 3H, $C\underline{CH_3}$)
1.61 (s, 6H, $2C\underline{CH_3}$)
Mass Spec (FAB⁻+ ions) m/e 547 $(M+K)^+$ 509 $(M^+ + H)$
Anal Calcd for $C_{17}H_{29}O_6P_2K_3$ (M.W.=508): C, 40.16; H, 5.71; P, 12.20
Found: C, 41.62; H, 6.60; P, 12.81
(Sample was dried to constant weight at 50° C).

## Example 4

(E,E)-(Hydroxy-5,9,13-trimethyl-4,8,12-pentadecatrienyl)oxy]phosphinyl]methyl]phosphonic acid, tripotassium salt

### A. (E,E)-3,7,11-Trimethyl-2,6,10-dodecatrienyl bromide

A solution of 1.00 g (4.5 mmol) of E,E-farnesol (Aldrich, further purified by flash chromatography) in 10 ml of distilled ether at 0° C under argon in the dark was treated dropwise with a solution of 195 µL (2.05 mmol, 0.45 eq.) of $PBr_3$ in 2 ml of ether. The resultant mixture was stirred at 0° C for one hour, then quenched with water and separated. The organic phase was washed with 5 ml of $H_2O$, 5 ml of saturated $NaHCO_3$, and 5 ml of brine, dried over $Na_2SO_4$ and evaporated to give 1.26 g (98%) of crude bromide as a clear oil.

TLC Silica (2:8 ethyl acetate:Hexane) Rf=0.69
$^1$H NMR ($CDCl_3$; 270 MHz)
δ 5.52 (t, 1H, J = 8.5 Hz)
5.08 (m, 2H)
4.01 (d, 2H, J=8.5 Hz)
1.9-2.2 (m, 8H)
1.73 (s, 3H)
1.68 (s, 3H)
1.60 (s, 6H) ppm.

### B. (E,E)-5,9,13-Trimethyl-4,8,12-tetradecatrienoic acid, 1,1-dimethylethyl ester

To a solution of 9.60 ml (68.5 mmol, 1.5 eq.) of diisopropylamine in 100 ml of THF at -78° C under argon was added 28.2 ml (45.0 mmol, 1.0 eq.) of 1.6M nBuLi in hexanes over 20 minutes. After warming to 0° C for 15 minutes, the solution was recooled to -78° C and 6.05 ml (45 mmol, 1.0 eq.) of t-butyl acetate was added over 20 minutes. After an additional 15 minutes, 16.0 ml (92 mmol, 2.05 eq.) of hexamethyl phosphoric triamide (HMPA) was added, followed by a solution of 12.53 g (45.0 mmol) of Part A farnesyl

bromide in 100 ml of THF over 20 minutes. The reaction was stirred at -78° C for 2.5 hours, quenched with saturated $NH_4Cl$, and allowed to warm to room temperature. After diluting with 400 ml of ethyl acetate, the mixture was washed with four 100 ml portions of water, and 200 ml of brine, dried over $MgSO_4$ and evaporated to provide 12.96 g of crude product as a yellow oil. Purification by flash chromatography on 1000 g of silica gel, eluted with 1:9 ethyl acetate:petroleum ether afforded 9.39 g (65%) of title compound as a pale yellow oil.

TLC Silica gel (2:98 ethyl acetate:hexane) Rf = 0.16

IR(neat) 2977, 2925, 2857, 1733, 1452, 1368, 1258, 1149 $cm^{-1}$.

$^1H$ NMR ($CDCl_3$, 270 MHz)

δ 5.10 (m, 3H)

2.25 (m, 4H)

1.9-2.1 (m, 8H)

1.68 (s, 3H)

1.62 (s, 3H)

1.59 (s, 6H)

1.44 (s, 9H) ppm.

Mass Spec (CI-$CH_4$/$N_2O$)(+ions) m/e 265 (M+H-$C_4H_8$), 247, 183, 137, 68, 57.

(- ions) m/e 319 (M-H), 279, 251, 100.

C. 5,9,13-Trimethyl-4,8,12-tetradecatrien-1-ol

A suspension of lithium aluminum hydride (1.28 mmol, 0.048 g) in 0.650 ml of dry ethyl ether was stirred under argon and cooled to 0° C. This suspension was treated dropwise with a solution of the Part B t-butyl ester (1.28 mmol, 0.408 g) in 0.650 ml of dry ethyl ether. The homogeneous reaction mixture was warmed to room temperature, stirred for 30 minutes, and then recooled to 0° C and quenched via dropwise addition of 0.048 ml of $H_2O$, followed by 0.048 ml of 1N NaOH, and finally 0.144 ml of $H_2O$. This suspension was allowed to stir at room temperature for 30 minutes. The suspension was diluted with ethyl ether, filtered, and the aluminum salts washed with ethyl ether. The filtrate was evaporated. The colorless residue was dissolved in 40% ethyl acetate/hexane and eluted in this solvent as a 2"/min flow rate through a 40 mm diameter flash chromatography column containing 6" of Merck silica gel. This purification provided 0.298 g (1.19 mmol) 93% yield of the title alcohol as a clear oil.

TLC:Silica gel Rf = 0.35 (50% ethyl acetate/hexane)

IR(film) 3330, 2960, 2920, 2850, 1715, 1665, 1440, 1380, 1150, 1105, 1060 $cm^{-1}$

Mass Spec (CI) m/e 251 $(M+H)^+$

$^1H$-NMR (270 MHz, $CDCl_3$)

δ 5.20-5.06 (m, 3H)

3.65 (t, 2H, J = 5.40 Hz)

2.10-1.93 (m, 12H)

1.70 (s, 3H)

1.68-1.60 (2 x s, 9H)

$^{13}C$ NMR (67.8 MHz, $CDCl_3$)

δ 135.73, 134.96, 131.18, 124.37, 124.09, 123.73, 62.63, 39.70, 32.72, 26.72, 26.56, 25.64, 24.24, 17.63, 15.96

D. 5,9,13-Trimethyl-4,8,12-tetradecatrien-1-ol, 4-(methylphenyl)sulfonate

A solution of Part C bis-homofarnesol (1.16 mmol, 0.290 g) in 3.0 ml of dry $CH_2Cl_2$ was stirred under argon at room temperature. The following were added in one portion: pyridine (5.79 mmol, 0.458 g, 0.468 ml), p-toluenesulfonyl chloride (2.55 mmol, 0.486 g), and finally dimethylaminopyridine (DMAP) (0.232 mmol, 0.028 g). This reaction mixture was stirred at room temperature for 6.5 hours. The reaction mixture was quenched with 4 ml of saturated aqueous $NaHCO_3$ and stirred vigorously for 40 minutes. The reaction mixture was then diluted with $H_2O$ and the aqueous layer extracted with $CH_2Cl_2$. The organic extracts were combined and washed twice with 5% $CuSO_4$ (aqueous) followed by an $H_2O$ wash. The resulting organic extract was dried over $MgSO_4$, filtered and evaporated to provide a green oil which was purified via flash chromatography (40 mm diameter columm, 6" Merck silica gel, 7% ethyl acetate/hexane eluent, 2"/min flow rate) to afford 0.375 g (0.928 mmol) 80% yield of the title tosylate as a pale yellow oil.

TLC:Silica gel Rf = 0.53 (20% ethyl acetate/hexane)
IR (film) 2970, 2920, 2860, 1600, 1445, 1365, 1190, 1180, 1100, 970, 935, 840, 815 $cm^{-1}$
Mass Spec (CI) m/e 405 $(M+H)^+$
$^1$H NMR (270 MHz, $CDCl_3$)
δ 7.80 (d, 2H, J = 6.0 Hz)
7.35 (d, 2H, J = 6.0 Hz)
5.08 (m, 2H)
4.98 (m, 1H)
4.03 (t, 2H, J = 5.8 Hz)
2.47 (s, 3H)
2.11-1.97 (m, 10H)
1.73-1.68 (m, 2H)
1.70 (s, 3H)
1.61 (s, 6H)
1.58 (s, 3H)
$^{13}$C NMR (67.8 MHz, $CDCl_3$)
δ 144.55, 136.79, 135.03, 133.33, 131.21, 129.73, 127.86, 124.32, 124.01, 122.20, 70.05, 39.67, 39.61, 28.96, 26.75, 26.53, 25.66, 23.63, 21.56, 17.66, 15.96.

E. (E,E)-(Hydroxy-5,9,13-trimethyl-4,8,12-pentadecatrientyl)oxy]phosphinyl]methyl]phosphonic acid, tripotassium salt

Tris (tetra-n-butylammonium)methylene bisphosphonate (prepared as described in Example 3) (1.76 mmol, 1.58 g) was stirred under argon at room temperature in 1.76 ml of dry $CH_3CN$ and treated dropwise (quickly) with a solution of the Part D tosylate (0.838 mmol, 0.339 g) in 1.67 ml of dry $CH_3CN$. The reaction mixture was stirred at room temperature for 4 hours. The $CH_3CN$ was removed *in vacuo* and the dark golden residue was dissolved in 3 ml of distilled $H_2O$ and eluted through an ion exchange column (AG 50W-X8, 100-200 mesh, 45 ml/76.5 meq of resin in a 20 mm diameter column). The columm was eluted with 1:49 i-$C_3H_7OH/H_2O$, and product fractions combined and lyophilized to provide 0.910 g of a yellow powdery solid. The lyophilate was again eluted through an ion exchange columm as above (60 ml, 102 meq of resin). Lyophilization of product fractions afforded 0.812 g of a yellow solid (no change in TLC). The desired pure product was obtained by dissolving the lyophilate in 3 ml of distilled $H_2O$ and eluting through an HP-20 chromatography column (2.5 cm x 15 cm of resin). Elution with 250 ml of $H_2O$, followed by an acetonitrile/$H_2O$ gradient, collecting 10 ml fractions every 1.4 minutes afforded (after lyophilization and drying under high vacuum over $P_2O_5$), 0.274 g (0.524 mmol), 63% yield of title product in the form of a white lyophilate.
TLC:Silica gel Rf = 0.46 (6:3:1/n-$C_3H_7OH$:con $NH_3$:$H_2O$)
IR(KBr) 3030, 2966, 2925, 2857, 1636, 1206, 1088, 1048, 971, 801, 519 $cm^{-1}$
Mass Spec (FAB) m/e 523 $(M+H)^+$
561 $(M+K)^+$.
$^1$H-NMR (400 MHz, $D_2O$)
δ 5.24 (m, 1H)
5.18 (m, 2H)
3.89-3.84 (m, 2H)
2.16-2.00 (m, 12H)
1.68 (s, 3H)
1.68-1.66 (m, 2H)
1.63 (s, 3H)
1.61 (s, 3H)
$^{13}$C NMR (67.8 MHz, $D_2O$, dioxane reference) δ 137.60, 136.82, 133.56, 125.46, 124.99, 65.56, 65.48, 40.17, 40.09, 31.64, 31.55, 30.47, 28.68, 27.12, 26.84, 26.06, 24.80, 18.14, 16.46

Anal Calcd for $C_{18}H_{32}O_6P_2K_2$ M.W. = 484.574:
C, 44.61; H, 6.66; P, 12.78
Found: C, 45.27; H, 6.81; P, 13.52

Sample was dried at 50° C for 6 hours.

Example 5

(E,E)-[[Hydroxy[(6,10,14-trimethyl-5,9,13-pentadecatrienyl)oxy]phosphinyl]methyl]phosphonic acid, tripotassium salt

A. 1-Chloro-3,7,11-trimethyl-2,6,10-dodecatriene

(Note: all temperatures indicated are for the contents of the reaction flask). To a stirred solution of 299 mg (2.24 mmol) of N-chlorosuccinimide in 15 ml of dichloromethane at -30° C under argon was added 0.18 ml (2.45 mmol) of distilled dimethyl sulfide over 5 minutes. After 10 minutes at -30° C, the reaction was allowed to warm to 0° C for 10 minutes, followed by cooling to -40° C. A solution of 441.4 mg (1.99 mmol) of 3,7,11-trimethyl-2,6,10-tridecatrien-1-ol in 5 ml of dichloromethane was added dropwise over 10 minutes. The reaction was allowed to warm gradually to 0° C over 1 hour, and then maintained at 0° C for 1 hour. After quenching with cold water, the mixture was extracted with hexane and the hexane extract was washed with cold water and cold brine, dried ($MgSO_4$) and evaporated to afford 483 mg of a crude product. Rapid flash chromatography on 20 g of Merck 9385 silica gel eluted with 3:97 ethyl acetate:pet ether provided 406.5 mg (85%) of a colorless liquid. $^{13}C$ NMR indicated that this material contained a trace (3%) impurity.
TLC:Silica gel (2:98 ethyl acetate:hexane) Rf = 0.56
$^1H$ NMR($CDCl_3$) (270 MHz)
δ 5.44 (t, 1, J = 7.9 Hz)
5.09 (t, 2, J = 5.8 Hz)
4.07 (d, 2, J = 7.9 Hz)
1.9-2.2 (m, 9)
1.72 (s, 3)
1.68 (s, 3)
1.60 (s, 6) ppm.

B. Dichloro[mu-[1-propanolato(2-)-$C^3$:$O^1$]]dimagnesium

A modification of the procedure of G. Cahiez et al. was employed (Tetrahedron Letters, 1978, 3013-4): To a stirred solution of 1.89 g (20 mmol) of 3-chloropropanol in 20 ml of THF under argon at -20° C was added 10 ml (20 mmol) of 2 M phenylmagnesium chloride in THF over 15 minutes. After 10 minutes at -20° C, the reaction was allowed to warm to room temperature, 730 mg (30 mmol) of magnesium turnings were added and the reaction was heated to reflux. Two 40 µl portions of 1,2-dibromoethane were added, the first portion injected at the start of reflux, and the second after 1 hour. After refluxing for a total of 2 hours, the reaction was allowed to cool to room temperature and was diluted with 37 ml of THF for a theoretical concentration of 0.3 M.

C. 6,10,14-Trimethyl-5,9,13-pentadecatrien-1-ol

A solution of 37.5 mL (20.3 mmol, 5.1 eq.) of a 0.54 M solution of Grignard reagent (Part B) in tetrahydrofuran and 9 mL of hexamethylphosphoramide at room temperature under argon was treated over 10 minutes with a solution of 955.5 mg (3.97 mmol) of farnesyl chloride (Part A) in 5 mL of tetrahydrofuran. After one hour, the reaction mixture was diluted with a mixture of 1:1 diethyl ether : hexane and quenched with 1 M HCl. The organic phase was washed with three 25 mL portions of saturated $NaHCO_3$, three 25 mL portions of $H_2O$, and 25 mL of brine, dried over $MgSO_4$ and evaporated to obtain 995.0 mg of crude product. Purification required two chromatographies. The first was run on 70 g of silica gel, eluting with 1:99 ethyl acetate:$CH_2Cl_2$ to provide 484.3 mg of impure material and 307.7 mg of pure title compound. The second chromatography, of the impure fractions, on 50 g of silica gel eluted with 0.75:99.25 ethyl acetate:$CH_2Cl_2$ gave 117.2 mg of slightly impure material and 302.8 mg of pure title compound. Combination of pure material from both columns gave a yield a yield of 610.5 mg (58%) of pure desired title isomer.
TLC:Silia gel (10:90 ethyl ether:$CH_2Cl_2$) Rf = 0.38
IR ($CCl_4$) 3639, 3450, 2964, 2930, 2858, 1449, 1382, 1058, 1028, 776, 750 $cm^{-1}$.
$^1H$ NMR ($CDCl_3$) (270 MHz)
δ 5.10 (m, 3H)
3.62 (t, 2H, J = 6.5 Hz)
2.00 (m, 10H)
1.69 (s, 3H)
1.61 (s, 9H)
1.2-1.7 (m, 5H, OH) ppm. Mass Spec (CI-$CH_4$/$N_2O$, + ions) m/e 282 (M + $NH_4$), 265 (M + H), 263 (m + H-$H_2$).

D. 6,10,14-Trimethyl-5,9,13-pentadecatrien-1-ol, 4-methylbenzenesulfonate

Following the procedure of Example 4 Part D, except substituting the above Part C alcohol for the Example 4 Part C alcohol, the title tosylate is obtained.

E. (E,E)-[[Hydroxy[(6,10,14-trimethyl-5,9,13-pentadecatrienyl)oxy]phosphinyl]methyl]phosphonic acid, tripotassium salt

Following the procedure of Example 4 Part E except substituting the above Part D tosylate for the Example 4 Part D tosylate, the title compound is obtained.

Examples 6 to 22

Following the procedures of Examples 1 to 5, the following additional compounds may be prepared in accordance with the present invention.

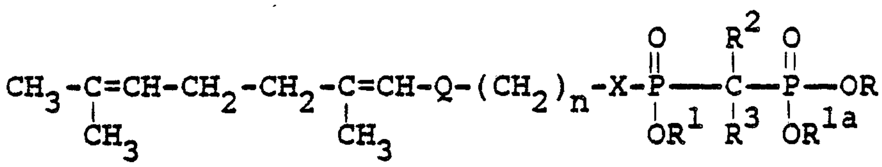

| Ex. No. | Q | X | n | $R^2$ | $R^3$ | R | $R^1$ | $R^{1a}$ |
|---|---|---|---|---|---|---|---|---|
| 6. | 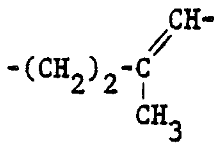 | O | 2 | H | H | K | K | K |
| 7. | bond | O | 3 | F | F | Na | Na | Na |
| 8. | 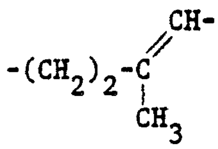 | -NH- | 1 | Cl | Cl | Na | Na | Na |
| 9. | 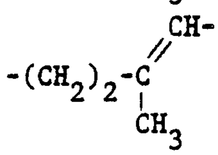 | -N-<br>$CH_3$ | 2 | H | Cl | Na | Na | Na |
| 10. | 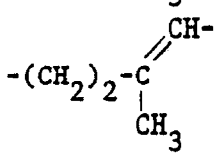 | O | 2 | F | H | K | K | K |
| 11. | 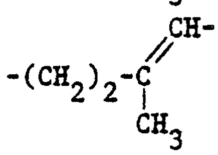 | -NH- | 4 | H | H | $CH_3$ | K | K |
| 12. | 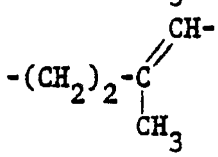 | $-NC_2H_5-$ | 3 | H | F | Na | Na | Na |
| 13. | bond | O | 4 | H | H | K | K | K |
| 14. | 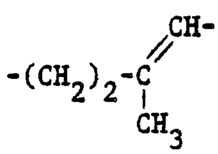 | O | 3 | F | H | H | Mg | Mg |

| Ex. No. | Q | X | n | $R^2$ | $R^3$ | R | $R^1$ | $R^{1a}$ |
|---|---|---|---|---|---|---|---|---|
| 15. | 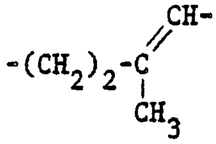 | -NH- | 2 | Cl | Cl | K | K | K |
| 16. | 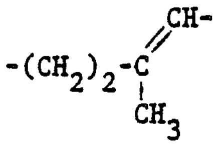 | -NH- | 1 | H | H | K | K | K |
| 17. | 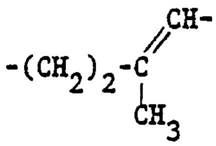 | O | 2 | F | F | K | K | K |
| 18. | 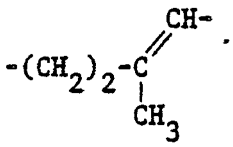 | O | 3 | F | F | K | K | K |
| 19. | 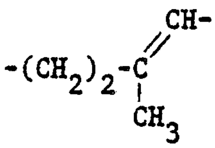 | O | 4 | F | F | K | K | K |
| 20. | bond | O | 4 | F | F | K | K | K |
| 21. | 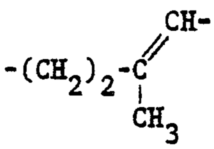 | NH | 1 | F | F | K | K | K |
| 22. | bond | NH | 1 | H | H | K | K | K |

## Claims

1. A compound having the structure

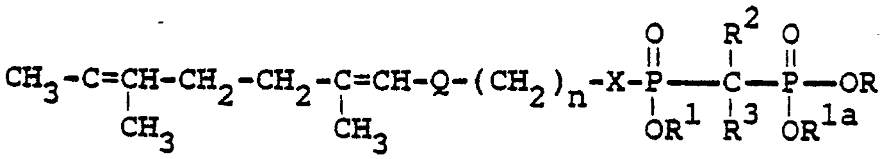

wherein Q is

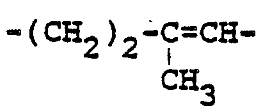

or a bond;
n is 1 to 4;
X is O, -NH- or $-NR^4-$;
R, $R^1$ and $R^{1a}$ are the same or different and are H, lower alkyl, lower alkenyl, or a metal ion;

$R^2$ and $R^3$ may be the same or different and are H or halogen; and
$R^4$ is lower alkyl;
with the proviso that when X is O, n is 2, 3, or 4.

2. The compound as defined in Claim 1 having the structure

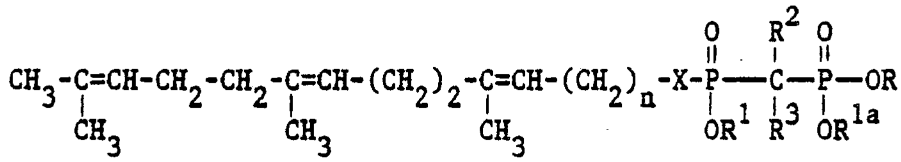

3. The compound as defined in Claim 1 having the structure

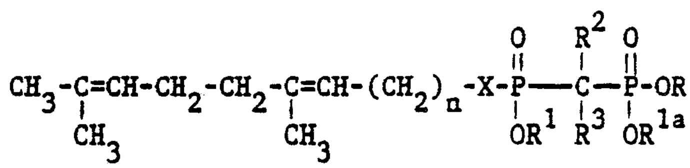

4. The compound as defined in Claim 1 having the structure

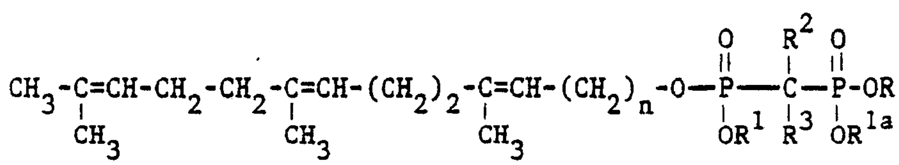

wherein n is 2, 3 or 4.

5. The compound as defined in Claim 1 having the structure

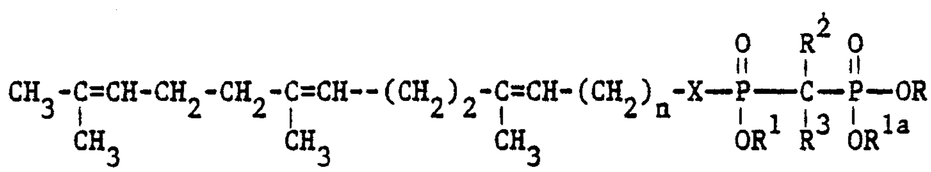

wherein X is -NH- or -$NR^4$-.

6. The compound as defined in Claim 1 wherein $R^2$ and $R^3$ are each H.

7. The compound as defined in Claim 1 wherein $R^2$ and $R^3$ are each halogen.

8. The compound as defined in Claim 1 having the name (E,E)-[[methoxy[(3,7,11-trimethyl-2,6,10-dodecatrienyl)amino]phosphinyl]methyl]phosphonic acid, dimethyl ester.

9. The compound as defined in Claim 1 having the name (E,E)-[[hydroxy[(3,7,11-trimethyl-2,6,10-dodecatrienyl)amino]phosphinyl]methyl]phosphonic acid or the trisodium salt, or its methyl ester, or its disodium salt.

10. The compound as defined in Claim 1 having the name (E,E)-[[hydroxy[(4,8,12-trimethyl-3,7,11-tridecatrienyl)oxy]phosphinyl]methyl]phosphonic acid, or its tri-potassium salts.

11. The compound as defined in Claim 1 having the name (E,E)-[[hydroxy[(5,9,13-trimethyl-4,8,12-tetradecatrienyl)oxy]phosphinyl]methyl]phosphonic acid, or its tripotassium salt.

12. The compound as defined in Claim 1 having the name (E,E)-[[hydroxy(6,10,14-trimethyl-5,9,13-pentadecatrienyl)oxy]phosphinyl]methyl]phosphonic acid, or its tripotassium salt.

13. A hypocholesterolemic or hypolipemic composition comprising a compound as defined in Claim 1 and a pharmaceutically acceptable carrier therefor.

14. Use of the compound as defined in Claim 1 for preparing a medicament for inhibiting cholesterol biosynthesis.